# EUROPÄISCHE PATENTANMELDUNG

(11) **EP 2 382 960 A1**
(43) Veröffentlichungstag der Anmeldung: **02.11.2011**
(21) Anmeldenummer: 10004140.9
(22) Anmeldetag: 19.04.2010
(51) Int. Cl.: A61K 6/04, A61L 27/30, A61L 27/54

(54) **Implantat mit antimikrobieller Beschichtung**

(71) Anmelder: DERU GmbH Entwicklung von medizinischen Produkten, 22844 Norderstedt (DE)
(72) Erfinder: Thull, Roger, 97082 Würzburg (DE)
(74) Vertreter: Glawe, Delfs, Moll

(57) **Zusammenfassung**

Die Erfindung betrifft ein Implantat mit einer Beschichtung (23), die im menschlichen Körper Silberionen abgibt und dadurch antimikrobiell wirkt. Erfindungsgemäß ist ein erster Oberflächenanteil der Beschichtung (23) von einem Anodenmaterial (25) gebildet. Ein zweiter Oberflächenanteil der Beschichtung (23) ist von einem Kathodenmaterial (26) gebildet. Das Kathodenmaterial steht in der elektrochemischen Spannungsreihe höher als das Anodenmaterial (25). Das Kathodenmaterial (26) und das Anodenmaterial (25) sind elektrisch leitend miteinander verbunden. Zusammen mit dem Körperelektrolyt in der Umgebung des Implantats bilden das Anodenmaterial (25) und das Kathodenmaterial (26) eine Vielzahl lokaler galvanischer Elemente. Die antimikrobielle Wirkung der Beschichtung (23) wird dadurch verbessert.

## Beschreibung

Die Erfindung betrifft ein Implantat mit einer Beschichtung, die im menschlichen Körper Silberionen abgibt und dadurch eine antimikrobielle Wirkung hat.

Wenn Implantate in den menschlichen Körper eingesetzt werden, besteht das Risiko von Infektionen. Auslöser der Infektionen können Mikroorganismen sein, die mit dem Implantat in den menschlichen Körper eingebracht werden oder die auf der Oberfläche des Implantats sitzen. Es ist bekannt, dass das Risiko von Infektionen vermindert werden kann, indem das Implantat mit einer Beschichtung versehen wird, die Silberionen an ihre Umgebung abgibt. Die Silberionen haben bekanntermaßen eine antimikrobielle Wirkung. Sie haben zudem den Vorteil, dass sie - wenn sie nicht auf einen Mikroorganismus treffen und gegen diesen wirken - sich mit dem Chlorid des Körperelektrolyts zu AgCl verbinden und in dieser Form aus dem Körper ausgeschieden werden können. Anders als andere antimikrobiell wirksame Stoffe reichern sich die Silberionen also nicht im Körper an.

Die bekannten Silberbeschichtungen geben nur in begrenztem Umfang Silberionen ab. Die abgegebenen Silberionen bewegen sich zudem zufällig in der Umgebung des Implantats. Es besteht deswegen eine hohe Wahrscheinlichkeit, dass die Silberionen im Körperelektrolyt zu AgCl kombinieren und dadurch ihre antimikrobielle Wirksamkeit verlieren, bevor sie auf einen Mikroorganismus treffen.

Der Erfindung liegt die Aufgabe zu Grunde, ein Implantat vorzustellen, dessen Beschichtung eine verbesserte antimikrobielle Wirksamkeit hat. Ausgehend vom eingangs genannten Stand der Technik wird die Aufgabe gelöst durch die Merkmale des Anspruchs 1. Vorteilhafte Ausführungsformen finden sich in den Unteransprüchen.

Erfindungsgemäß wird ein erster Oberflächenanteil der Beschichtung von einem silberhaltigen Anodenmaterial gebildet, das zur Abgabe von Silberionen vorgesehen ist. Für einen zweiten Oberflächenanteil ist ein Kathodenmaterial vorgesehen. Das Kathodenmaterial steht in der elektrochemischen Spannungsreihe höher als das Anodenmaterial. Das Kathodenmaterial und das Anodenmaterial sind elektrisch leitend miteinander verbunden.

Zunächst werden einige Begriffe erläutert. Der Begriff Implantat umfasst alle Arten von in den Körper einzusetzenden Gegenständen. Dies sind beispielsweise Endoprothesen für Knochen oder Gelenke aber auch Implantate, die in anderen Arten von Körpergewebe eingesetzt werden, wie beispielsweise Stents im Herz-Kreislaufsystem. Umfasst sind auch Implantate, die nur zum Teil in den menschlichen Körper aufgenommen werden und zum Teil herausragen, wie Zahnimplantate oder externe Fixateure, die eine indirekte, teilweise mit einer Spannvorrichtung außerhalb des Körpers stattfindende stabilisierende Osteosynthese darstellen.

Mit dem Begriff erster und zweiter Oberflächenanteil wird zum Ausdruck gebracht, dass das Kathodenmaterial in der Beschichtung räumlich von dem Anodenmaterial getrennt ist. Nicht gemeint ist also eine Beschichtung, in der mehrere Materialien gleichmäßig miteinander vermischt sind. Möglich, aber nicht zwingend erforderlich ist es, dass der zweite Oberflächenanteil flächendeckend mit dem Kathodenmaterial belegt ist.

In der elektrochemischen Spannungsreihe sind die Stoffe nach ihrem Standardelektrodenpotential sortiert. Je höher die Stellung eines Stoffs in der elektrochemischen Spannungsreihe ist, desto geringer ist sein Lösungsdruck, also sein Bestreben, Ionen an in der Umgebung befindliches Wasser abzugeben. Ein Metall, das in der elektrochemischen Spannungsreihe höher steht, wird als edel bezeichnet; ein Metall, das in der elektrochemischen Spannungsreihe niedriger steht, wird als unedel bezeichnet. Für die meisten Stoffe ist die Stellung in der elektrochemischen Spannungsreihe bekannt, der betreffende Wert kann den einschlägigen Tabellen entnommen werden. Ist die Stellung eines Stoffs in der elektrochemischen Spannungsreihe nicht bekannt, kann sie bestimmt werden, indem ein galvanisches Element mit einem bekannten Stoff aufgebaut wird und die entstehende Potenzialdifferenz gemessen wird. Anhand der Potenzialdifferenz kann die Stellung in der Spannungsreihe bestimmt werden. Die Begriffe Anodenmaterial und Kathodenmaterial dienen dazu, die relative Stellung der verwendeten Materialien zueinander in der elektrochemischen Spannungsreihe darzustellen. Das Anodenmaterial und das Kathodenmaterial sind elektrisch leitende Materialien.

Wenn das Implantat in den Körper eingesetzt wird, bilden das Anodenmaterial und das Kathodenmaterial der Beschichtung mit dem in der Umgebung des Implantats befindlichen Körperelektrolyt ein lokales galvanisches Element. Die Neigung des Anodenmaterials, Silberionen an die Umgebung abzugeben, wird dadurch verstärkt. Die Elektronen, die nach der Abgabe der Silberionen in dem Anodenmaterial zurückbleiben, können sich aufgrund der elektrischen Verbindung in das Kathodenmaterial bewegen. Durch die Potenzialdifferenz werden die Silberionen in Richtung des Kathodenmaterials angezogen.

Die Wirkung der erfindungsgemäßen Beschichtung ist also eine doppelte. Erstens hat das Anodenmaterial aufgrund des lokalen galvanischen Elements eine stärkere Neigung, Silberionen an das umgebende Körperelektrolyt abzugeben. Verglichen mit einer Beschichtung, die nur aus dem betreffenden Anodenmaterial besteht, wird also eine größere Anzahl von Silberionen abgegeben, wodurch sich die antimikrobielle Wirksamkeit erhöht. Außerdem verläuft die Bewegung der abgegebenen Silberionen nicht mehr in beliebigen Richtungen, sondern die Silberionen werden in Richtung der Potentialdifferenz zwischen den beiden Werkstoffen, also in Richtung des Kathodenmaterials bewegt. Die Wahrscheinlichkeit ist erhöht, dass die Silberionen tatsächlich gegen auf der Oberfläche des Implantats sitzende Mikroorganismen wirksam werden, anstatt im Körperelektrolyt zu AgCl zu kombinieren und die antimikrobielle Wirksamkeit dadurch zu verlieren. Die Wirkung der erfindungsgemäßen Beschichtung ist also auf die Oberfläche des Implantats konzentriert. Die Beschichtung ist besonders geeignet zur Bekämpfung des gefährlichen Biofilms, der sich auf der Oberfläche von Implantaten bilden kann.

Die Beschichtung kann die gesamte Oberfläche des Implantats bedecken. Dies wird sich bei vielen Implantaten anbieten, die insgesamt in den Körper eingesetzt werden. Insbesondere bei Gelenkendoprothesen kann auch vorgesehen sein, dass nur ein Teil der Oberfläche beschichtet ist. Die Beschichtung kann auf den Teil der Oberfläche aufgebracht sein, mit dem die Prothese im implantierten Zustand mit Körpergewebe in Kontakt ist, während ein anderer Teil der Oberfläche, der beispielsweise zum Zusammenwirken mit einer anderen Prothesenkomponente bestimmt ist oder sich wie beim Fixateuer extern außerhalb des Körpers befindet, frei von der Beschichtung ist.

Das Anodenmaterial kann reines Silber sein. Mit einem Standardelektrodenpotential von ungefähr +0,8 V ist Silber ein relativ edles Metall, das zum oberen Bereich der elektrochemischen Spannungsreihe gehört. Bezugsgröße für die Spannungsangaben des Standardelektrodenpotentials ist die Normal-Wasserstoffelektrode.

Das mit dem reinen Silber zusammenwirkende Kathodenmaterial muss ein Standardelektrodenpotential von mehr als +0,8 V haben. Wenn das Kathodenmaterial ein Metall ist, ist es also edler als Silber. Ein zum Zusammenwirken mit reinem Silber geeignetes Kathodenmaterial ist beispielsweise Gold, das ein Standardelektrodenpotential in der Größenordnung von +1,5 V hat. Auch wenn nicht reines Silber, sondern eine Legierung aus Silber und einem anderen Stoff als Anodenmaterial verwendet wird, sollte das Standardelektrodenpotential des Kathodenmaterials größer als +0,8 V sein. Vorzugsweise ist das Standardelektrodenpotential des Kathodenmaterials um mindestens 0,3 V, weiter vorzugsweise um mindestens 0,5 V, weiter vorzugsweise mindestens 0,7 V höher als das Standardelektrodenpotential des Anodenmaterials.

Die Wirkung des lokalen galvanischen Elements ist desto stärker, je größer die Differenz zwischen dem Standardelektrodenpotential des Anodenmaterials und dem Standardelektrodenpotential des Kathodenmaterials ist. In einer vorteilhaften Ausführungsform wird als Anodenmaterial deswegen ein silberhaltiges Material verwendet, dessen Standardelektrodenpotential kleiner ist als +0,8 V. Das Anodenmaterial enthält dann außer den Silberbestandteilen, die sich aus der Anode herauslösen sollen, noch weitere Bestandteile. Das für das Anodenmaterial angegebene Standardelektrodenpotential bezieht sich auf den Lösungsdruck für Silberionen. Vorzugsweise wird für die Anode ein Material gewählt, aus dem heraus außer den Silberionen keine weiteren Stoffe an das Körperelektrolyt abgegeben werden. Wenn außer den Silberionen noch weitere Stoffe abgegeben werden, besteht das Risiko, dass die weiteren Stoffe im Körper unerwünschte Wirkungen haben. Sowohl für die Anode als auch für die Kathode sollte zudem ein Material ausgewählt werden, das biokompatibel ist.

Die antimikrobielle Wirkung der erfindungsgemäßen Beschichtung hängt an den Silberionen, die aus dem Kathodenmaterial heraus abgegeben werden. Die Anzahl der abgegebenen Silberionen wird größer, je größer der Oberflächenanteil der Beschichtung ist, der von dem Anodenmaterial eingenommen wird. Der von dem Anodenmaterial eingenommene Oberflächenanteil der Beschichtung ist deswegen vorzugsweise größer als 50%, weiter vorzugsweise größer als 70%, weiter vorzugsweise größer als 80%. Der von dem Kathodenmaterial eingenommene Flächenanteil ist im Vergleich von geringerer Bedeutung. Jedoch darf der Anteil des Kathodenmaterials, wenn eine gute Wirksamkeit der galvanischen Elemente erreicht werden soll, nicht zu klein sein. Vorzugsweise ist der Anteil des Kathodenmaterials an der Oberfläche der Beschichtung größer als 0,1%, weiter vorzugsweise größer als 1%, weiter vorzugsweise größer als 5%.

Es ist gewünscht, dass die Silberionen, nachdem sie aus dem Anodenmaterial ausgetreten sind, eine gewisse Strecke zurücklegen können, bevor sie auf das Kathodenmaterial treffen. Während dieser Bewegung können die Silberionen antimikrobiell wirken. Die von dem Anodenmaterial und dem Kathodenmaterial eingenommenen Oberflächenanteile der Beschichtung sollten deswegen so voneinander getrennt sein, dass die Silberionen nicht zwangsläufig unmittelbar auf das Kathodenmaterial treffen. Die Beschichtung umfasst deswegen vorzugsweise eine Vielzahl kreisförmiger Oberflächenbereiche mit einem Durchmesser von mehr als 1 µm, weiter vorzugsweise mehr als 5 µm, weiter vorzugsweise mehr als 15 µm, weiter vorzugsweise mehr als 50 µm, die ausschließlich von Anodenmaterial gebildet sind und frei von Kathodenmaterial sind. Andererseits ist es für die Wirksamkeit der Beschichtung auch nicht vorteilhaft, wenn die freie Wegstrecke der Silberionen zu lang ist. Der Durchmesser der kreisförmigen Oberflächenbereiche sollte deswegen kleiner als 5 mm, vorzugsweise kleiner als 1 mm, weiter vorzugsweise kleiner als 0,5 mm sein. Es werden vorzugsweise mehr als 30%, weiter vorzugsweise mehr als 50% der Oberfläche der Beschichtung von solchen Oberflächenanteilen eingenommen.

Im Zentrum eines solchen Bereichs austretende Silberionen müssen eine gewisse Strecke zurücklegen, bevor sie auf Kathodenmaterial treffen. Während sie die Strecke zurücklegen, können sie antimikrobiell wirken. Die von den Silberionen zurückzulegende freie Strecke kann sich am Durchmesser der Bakterien orientieren, der ebenfalls im µm-Bereich liegt. Es kann davon ausgegangen werden, dass die Silberionen sich auf einem bogenförmigen Weg bewegen und dass die größte Entfernung zur Oberfläche, die die Silberionen auf ihrem Weg haben, in einer ähnlichen Größenordnung liegt, wie die Strecke, die parallel zur Oberfläche zurückgelegt wird. Wenn also die zurückzulegende freie Wegstrecke ungefähr dem Durchmesser der Bakterien entspricht, wird erreicht, dass die Silberionen auf ihrem gesamten Weg gegen auf der Oberfläche sitzende Bakterien wirken können.

Die Beschichtung kann so gestaltet sein, dass das Kathodenmaterial inselförmig in das Anodenmaterial eingelassen ist oder inselförmig auf das Anodenmaterial aufgebracht ist. Das Kathodenmaterial kann selbst in Form zusammenhängender Flächenbereiche mit einem Durchmesser von beispielsweise einigen µm aufgebracht sein. Nicht ausgeschlossen ist es, dass das Kathodenmaterial auf dem zweiten Oberflächenbereich in Form einzelner Partikel aufgebracht ist, ohne dass das Anodenmaterial in diesem Bereich flächendeckend überzogen ist.

In vielen Fällen soll die Oberfläche des Implantats glatt sein. Dies kann erreicht werden, indem das Anodenmaterial und das Kathodenmaterial bündig miteinander abschließen. In einer alternativen Ausführungsform kann Kathodenmaterial gegenüber dem Anodenmaterial vorspringen. Die Silberionen bewegen sich dann in einem kleinen Abstand zur Oberfläche der Beschichtung, so dass eine gute Wirkung gegen Mikroorganismen in der direkten Umgebung der Beschichtung erzielt wird. Es bietet sich dazu an, zunächst das Anodenmaterial in einer gleichmäßigen Schichtdicke aufzutragen und anschließend in ausgewählten Bereichen Kathodenmaterial auf die Beschichtung aufzubringen. Die Schichtdicke des Anodenmaterials kann zwischen 100 nm und 10.000 nm, vorzugsweise zwischen 200 nm und 400 nm liegen. Dieser Bereich gilt insbesondere, wenn das Anodenmaterial reines Silber ist. Die Schichtdicke des auf das Anodenmaterials aufgebrachten Kathodenmaterials kann ebenfalls zwischen 100 nm und 10.000 nm, vorzugsweise zwischen 200 nm und 400 nm liegen.

Möglich ist es auch, zunächst eine Schicht des Kathodenmaterials flächendeckend aufzubringen. Auf das Kathodenmaterial kann eine Schicht Anodenmaterial gelegt werden, die Durchbrechungen aufweist, so dass das Kathodenmaterial durch das Anodenmaterial hindurch von außen zugänglich ist. Wird das Anodenmaterial mit einem Plasma-Beschichtungsverfahren aufgebracht, so können die Durchbrechungen dadurch erzeugt werden, dass beim Aufbringen der Schicht größere Bruchstücke mit einem Durchmesser von beispielsweise 20µm auf die Oberfläche gerichtet werden, die ein Stück aus der sich bildenden Schicht herausschlagen, siehe WO 2009/036846. Auch bei dieser Vorgehensweise liegt die Dicke der Schichten vorzugsweise zwischen 100 nm und 10.000 nm, weiter vorzugsweise zwischen 200 nm und 400 nm.

Die Erfindung wird nachfolgend unter Bezugnahme auf die beigefügten Zeichnungen anhand vorteilhafter Ausführungsformen beispielhaft beschrieben. Es zeigen:
- Fig. 1:: eine erste Ausführungsform eines erfindungsgemäßen Implantats;
- Fig. 2:: eine Komponente des Implantats aus Fig. 1;
- Fig. 3:: eine zweite Ausführungsform eines erfindungsgemäßen Implantats;
- Fig. 4:: einen Ausschnitt aus dem Körper eines erfindungsgemäßen Implantats mit Beschichtung;
- Fig. 5:: die Beschichtung aus Fig. 4 in Draufsicht;
- Fig. 6:: die Ansicht aus Fig. 4 bei einer anderen Ausführungsform der Erfindung;
- Fig. 7:: die Ansicht aus Fig. 5 bei der Ausführungsform gemäß Fig. 6;
- Fig. 8:: die Ansicht aus Fig. 4 bei einer weiteren Ausführungsform der Erfindung; und
- Fig. 9:: die Ansicht aus Fig. 5 bei einer weiteren Ausführungsform der Erfindung.

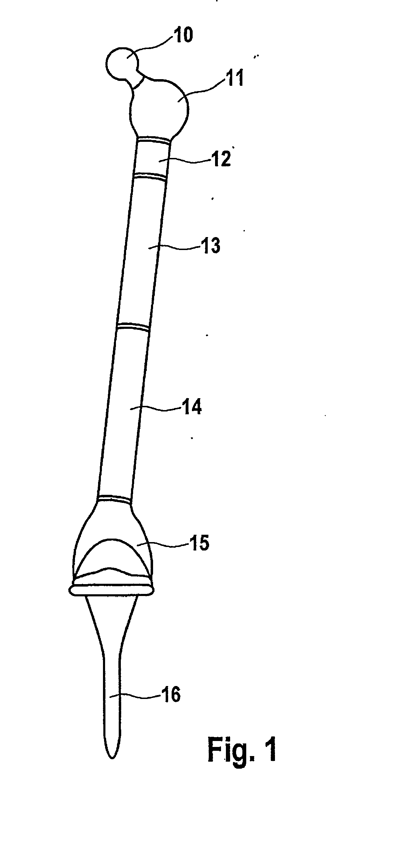

Ein in Fig. 1 gezeigtes Implantat ist dazu bestimmt, einen von der Hüfte bis unterhalb des Knies reichenden Teil des menschlichen Skeletts zu ersetzen. Ein kugelförmiger Gelenkkopf 10 bildet eine Gelenkfläche, die dazu ausgelegt ist, mit einem Acetabulum zusammenzuwirken. Der Gelenkkopf 10 ist mit einem Kopfstück 11 des Implantats über eine Schraubverbindung verbunden. Der den Mittelschaft des Oberschenkelknochens ersetzende Teil des Implantats umfasst drei Implantatkomponenten 12, 13, 14. Die Implantatkomponenten 12, 13, 14 sind untereinander und mit dem Kopfstück 11 ebenfalls über Schraubverbindungen verbunden. Ein Kniestück 15 bildet eine gelenkige Verbindung zu einem Schaft 16, der dazu bestimmt ist, das Implantat mit einem Unterschenkelknochen zu verbinden.

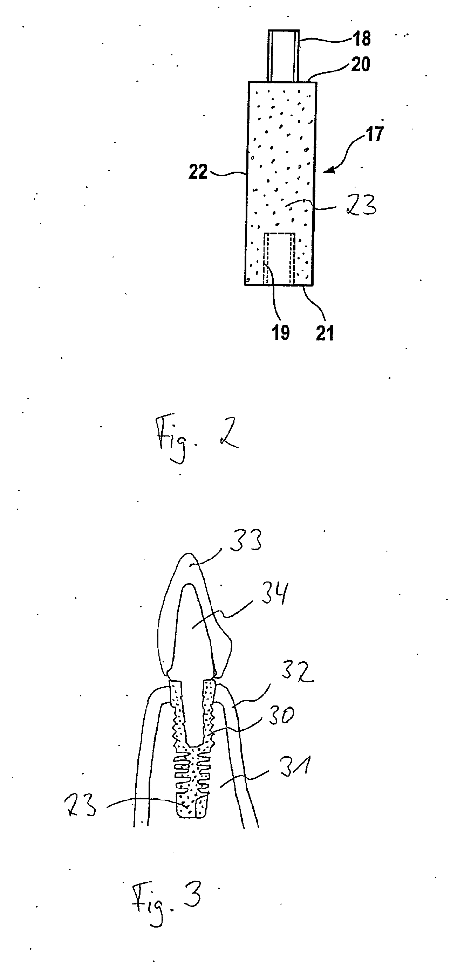

Die Implantatkomponenten 12, 13, 14 sind in unterschiedlichen Längen verfügbar, damit das Implantat an unterschiedlich lange Oberschenkelknochen angepasst werden kann. Die Fig. 2 zeigt eine den Implantatkomponenten 12, 13, 14 entsprechende Implantatkomponente 17 in vergrößerter Darstellung. Die Implantatkomponente 17 umfasst einen Schraubbolzen 18 sowie eine gestrichelt angedeutete Schraubbohrung 19. Über den Schraubbolzen 18 und die Schraubbohrung 19 kann die Implantatkomponente 17 an ihren beiden Enden mit weiteren Implantatkomponenten verbunden werden. Der Schraubbolzen 18, die Schraubbohrung 19 sowie die angrenzenden Stirnflächen 20 und 21 liegen im implantierten Zustand der Implantatkomponente 17 also nicht an Körpergewebe des Patienten an, sondern an anderen Implantatkomponenten. Die Mantelfläche 22 der Implantatkomponente 17 hingegen ist dazu ausgelegt, im implantierten Zustand Kontakt mit menschlichem Gewebe einzugehen. Die Mantelfläche 22 ist mit einer durch Punktierung angedeuteten antimikrobiellen Beschichtung 23 versehen. Die restliche Oberfläche der Implantatkomponente ist frei von der Beschichtung 23.

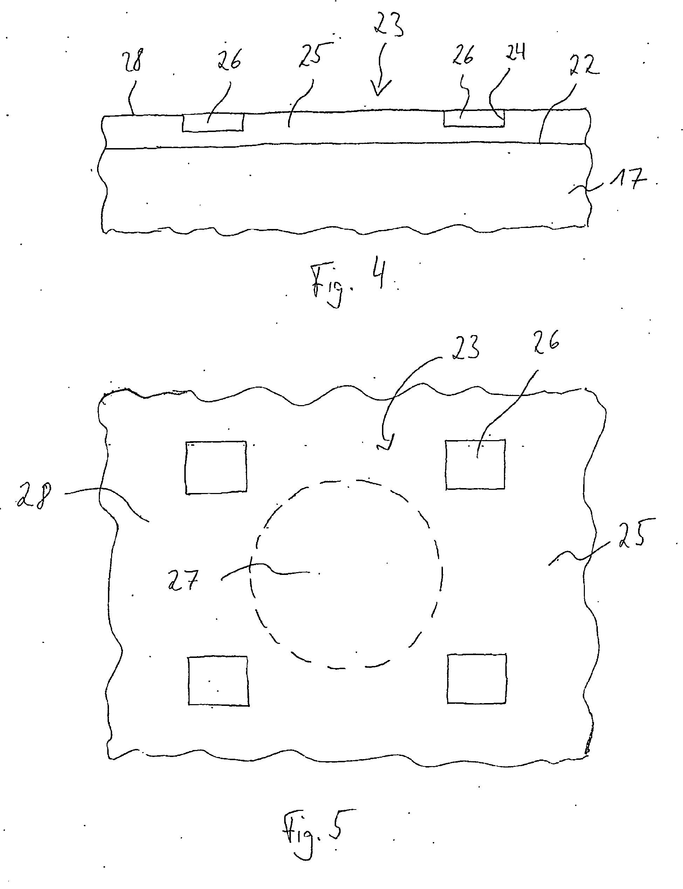

Die Beschichtung 23 ist in den Figuren 4 und 5 vergrößert dargestellt. Die Beschichtung 23 besteht zum größten Teil aus reinem Silber, das die Mantelfläche flächendeckend überzieht. In die Silberschicht ist, wie Fig. 5 zeigt, Goldmaterial in Form mehrerer rechteckiger Inseln eingebracht. Das Goldmaterial ist in die Silberschicht eingelassen, so dass die beiden Materialien bündig miteinander abschließen und eine glatte Oberfläche entsteht. Eine glatte Oberfläche ist erwünscht, weil das umgebende Körpergewebe möglichst wenig durch Reibung gereizt werden soll. Die Beschichtung 23 hat einen ersten Oberflächenanteil 28, der durch das Silbermaterial gebildet wird, und einen zweiten Oberflächenanteil 29, der durch das Goldmaterial gebildet wird. Der von dem Silbermaterial gebildete Oberflächenanteil 28 nimmt mehr als 80% der Oberfläche der Beschichtung 23 ein. Zwischen den Inseln bleiben, wie in Fig. 5 in gestrichelter Linie angedeutet ist, kreisförmige Oberflächenbereiche 27, in denen die Oberfläche der Beschichtung 23 vollständig aus Silbermaterial besteht und nicht durch Goldmaterial unterbrochen ist. Der Oberflächenbereich 27 hat einen Durchmesser von mehr als 0,1 mm.

Das Silber und das Gold sind in der Beschichtung 23 elektrisch leitend miteinander verbunden. Silber ist ein unedleres Metall als Gold und steht in der elektrochemischen Spannungsreihe tiefer als Gold. Im Sinne der erfindungsgemäßen Funktion der Beschichtung ist Silber also ein Anodenmaterial 25 und Gold ein Kathodenmaterial 26.

Nach der Implantation ist die Beschichtung 23 mit Körperelektrolyt umgeben. Das Silbermaterial hat eine Neigung, positiv geladene Silberionen an das Körperelektrolyt abzugeben. Diese Neigung wird als Lösungsdruck bezeichnet. Wenn Silberionen aus der Beschichtung heraus abgegeben werden, bleiben in der Beschichtung überschüssige Elektronen zurück und es bildet sich ein Überschuss an negativen Ladungsträgern in der Beschichtung. Da das Silbermaterial und das Goldmaterial elektrisch leitend miteinander verbunden sind, können die überschüssigen Elektronen sich frei in Richtung des Goldmaterials bewegen. Das Goldmaterial unterliegt ebenfalls einem gewissen Lösungsdruck, Ionen an das Körperelektrolyt abzugeben. Da Gold ein edleres Metall ist als Silber und in der elektrochemischen Spannungsreihe höher steht, ist der Lösungsdruck jedoch geringer als der Lösungsdruck des Silbers. Die in größerer Konzentration abgegebenen Silberionen bewegen sich zu dem Goldmaterial hin. Auf diese Weise bildet das Körperelektrolyt zusammen mit dem Silber als Anodenmaterial 25 und mit dem Gold als Kathodenmaterial 26 lokale galvanische Elemente. Die Silberionen treten aus dem Anodenmaterial 25 aus und bewegen sich parallel zu der Beschichtung 23 in Richtung des Kathodenmaterials 26. Auf diesem Weg können die Silberionen eine antimikrobielle Wirkung entfalten gegenüber Mikroorganismen, die auf der Oberfläche der Beschichtung 23 sitzen.

Das in Fig. 3 gezeigte Zahnimplantat ist eine alternative Ausführungsform der Erfindung. Ein Implantatkörper 30 ist mit seinem unteren Ende in den Kieferknochen 31 eingeschraubt. Das obere Ende des Implantatkörpers 30 ragt aus dem Kieferknochen 31 und dem den Kieferknochen 31 umgebenden Zahnfleisch 32 nach oben heraus. Ein mit einer künstlichen Zahnkrone 33 überzogener Aufbaupfosten 34 ist in das freie Ende des Implantatkörpers 30 eingeschraubt. Das Zahnimplantat ersetzt auf diese Weise einen natürlichen Zahn. Der Implantatkörper 30 ist wiederum mit einer durch Punktierung angedeuteten Beschichtung 23 versehen.

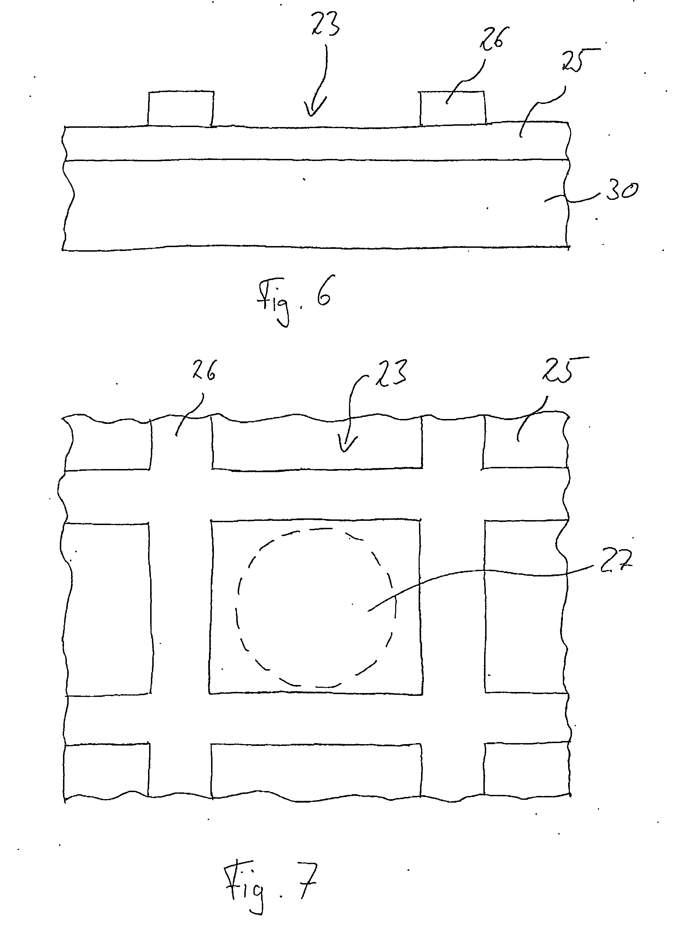

Die Beschichtung 23 ist in den Figuren 6 und 7 in vergrößerter Darstellung gezeigt. Auf die Oberfläche des Implantats 30 ist zunächst eine Silberbeschichtung aufgebracht, die eine gleichmäßige Dicke von ungefähr 400 nm hat. Auf die Oberfläche der Silberbeschichtung ist in gitterförmiger Anordnung Goldmaterial mit einer Schichtdicke von ebenfalls ungefähr 400 nm aufgetragen. Die in dem Gitter eingeschlossenen Bereiche, in denen die Oberfläche der Beschichtung 23 von dem Silbermaterial gebildet wird, sind in ihrer Gesamtheit der erste Oberflächenanteil 28 der Beschichtung 23. Die gitterförmige Anordnung des Goldmaterials bildet den zweiten Oberflächenanteil 29 der Beschichtung. Die Gitterform des Goldmaterials ist so bemessen, dass kreisförmige Oberflächenbereiche 27 mit einem Durchmesser von mehr als 50 µm frei von dem Goldmaterial bleiben.

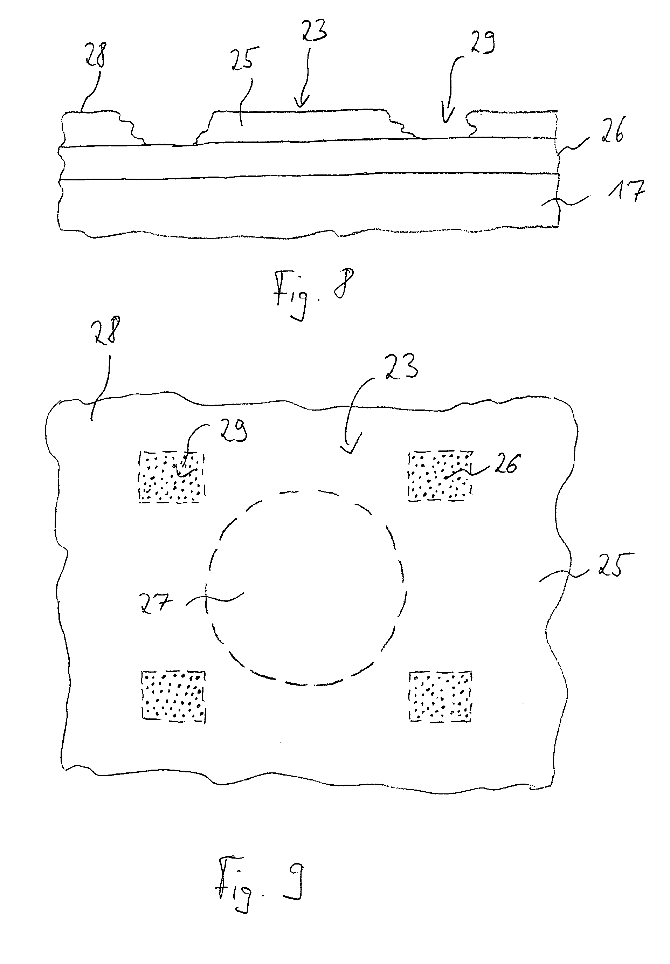

Bei der in Fig. 8 gezeigten Beschichtung ist die Implantatkomponente 17 zunächst flächendeckend mit einer Schicht aus aus Gold als Kathodenmaterial 26 überzogen. Eine darüber als Anodenmaterial 25 aufgebrachte Silberschicht weist eine Vielzahl von Durchbrechungen auf. Die Durchbrechungen bilden in ihrer Summe den zweiten Oberflächenanteil 29, in dem das Anodenmaterial 25, durch das Kathodenmaterial 26 hindurch von außen zugänglich ist.

In der Ausführungsform der Fig. 9 ist das Anodenmaterial 26 auf dem zweiten Oberflächenanteil 29 nicht flächendeckend, sondern als Vielzahl einzelner Partikel aufgebracht. An der erfindungsgemäßen Wirkung der Beschichtung ändert dies nichts.

Wie oben erläutert ist das Silber im Sinne der Erfindung ein Anodenmaterial 25 und das Gold ein Kathodenmaterial 26. Zusammen mit dem Körperelektrolyt in der Umgebung des Implantatkörpers 30 bildet die Beschichtung 23 eine Vielzahl lokaler galvanischer Elemente. Da das Gold als Kathodenmaterial 26 gegenüber dem Anodenmaterial 25 vorspringt, können sich die Silberionen auch in einem geringen Abstand zu der Silberschicht in Richtung des Kathodenmaterials 26 bewegen.

Bei dem Zahnimplantat hat die antimikrobielle Beschichtung 23 insbesondere die Funktion, gegen Mikroorganismen am Übergang zwischen dem Implantatkörper 30 und dem Zahnfleisch 32 bzw. dem Kieferknochen 31 zu wirken. In der Mundumgebung gibt es bekanntlich eine Vielzahl von Mikroorganismen und das Risiko einer Infektion im Umfeld des Implantatkörpers 30 ist hoch. Wenn durch die antimikrobielle Beschichtung 23 das Eindringen von Mikroorganismen zwischen dem Implantatkörper 30 und dem Zahnfleisch 32 unterbunden wird, können für den Patienten unangenehme Infektionen verhindert werden.

## Patentansprüche

1. Implantat mit einer Beschichtung (23), die im menschlichen Körper Silberionen abgibt und dadurch eine antimikrobielle Wirkung hat, **dadurch gekennzeichnet, dass** ein erster Oberflächenanteil (28) der Beschichtung (23), von einem silberhaltigen Anodenmaterial (25) gebildet ist, dass auf einem zweiten Oberflächenanteil (29) ein Kathodenmaterial (26) vorgesehen ist, wobei das Kathodenmaterial (26) in der elektrochemischen Spannungsreihe höher steht als das Anodenmaterial (25), und dass das Kathodenmaterial (26) und das Anodenmaterial (25) elektrisch leitend miteinander verbunden sind.

2. Implantat nach Anspruch 1, **dadurch gekennzeichnet, dass** das Anodenmaterial (25) reines Silber ist.

3. Implantat nach Anspruch 1, **dadurch gekennzeichnet, dass** das auf die Abgabe von Silberionen bezogene Standardelektrodenpotential des Anodenmaterials (25) kleiner ist als +0,8 V.

4. Implantat nach einem der Ansprüche 1 bis 3, **dadurch gekennzeichnet**, das Standardelektrodenpotential des Kathodenmaterials größer ist +0,8 V.

5. Implantat nach Anspruch 4, **dadurch gekennzeichnet, dass** das Kathodenmaterial (26) Gold ist.

6. Implantat nach einem der Ansprüche 1 bis 5, **dadurch gekennzeichnet, dass** das Standardelektrodenpotential des Kathodenmaterials (26) um mindestens 0,3 V, vorzugsweise um mindestens 0,5 V, weiter vorzugsweise mindestens 0,7 V höher ist als das Standardelektrodenpotential des Anodenmaterials (25).

7. Implantat nach einem der Ansprüche 1 bis 6, **dadurch gekennzeichnet, dass** das Kathodenmaterial (26) inselförmig in das Anodenmaterial (25) eingelassen ist.

8. Implantat nach einem der Ansprüche 1 bis 7, **dadurch gekennzeichnet, dass** der von dem Anodenmaterial (25) gebildete erste Oberflächenanteil (28) mehr als 50% ein, vorzugsweise mehr als 70%, weiter vorzugsweise mehr als 80% der Oberfläche der Beschichtung (23) ausmacht.

9. Implantat nach einem der Ansprüche 1 bis 8, **dadurch gekennzeichnet, dass** die Beschichtung (23) kreisförmige Oberflächenbereiche (27) mit einem Durchmesser von mehr als 0,1 mm, vorzugsweise mehr als 0,5 mm, weiter vorzugsweise mehr als 1 mm aufweist, die frei von Kathodenmaterial (26) sind.

10. Implantat nach einem der Ansprüche 1 bis 9, **dadurch gekennzeichnet, dass** das Anodenmaterial (25) und das Kathodenmaterial (26) bündig miteinander abschließen.

11. Implantat nach einem der Ansprüche 1 bis 10, **dadurch gekennzeichnet, dass** das Kathodenmaterial (26) gegenüber dem Anodenmaterial (25) vorspringt.
